# EUROPÄISCHE PATENTSCHRIFT

(11) **EP 2 152 340 B1**
(45) Veröffentlichungstag und Bekanntmachung des Hinweises auf die Patenterteilung: **31.07.2019**
(21) Anmeldenummer: 08733806.7
(22) Anmeldetag: 22.04.2008
(51) Int. Cl.: A61M 5/315

(54) **RÜCKDREHBARE DOSIERVORRICHTUNG FÜR EINE INJEKTIONSVORRICHTUNG**
REVERSELY ROTATABLE DOSING MECHANISM FOR AN INJECTION DEVICE
DISPOSITIF DE DOSAGE RÉVERSIBLE POUR UN SYSTÈME D'INJECTION

(30) Priorität: 23.04.2007 DE 102007019124
(43) Veröffentlichungstag der Anmeldung: 17.02.2010
(73) Patentinhaber: Tecpharma Licensing AG, 3401 Burgdorf (CH)
(72) Erfinder: MOSER, Ulrich, CH-3412 Heimiswil (CH); SCHRUL, Christian, CH-3421 Lyssach (CH); HIRSCHEL, Juerg, CH-5000 Aarau (CH); TSCHIRREN, Markus, CH-3422 Kirchberg (CH)
(86) Internationale Anmeldenummer: PCT/CH2008/000180
(87) Internationale Veröffentlichungsnummer: WO 2008/128373

(56) Entgegenhaltungen:
- EP-A- 0 295 075
- WO-A-2006/089734
- US-A- 5 298 023
- US-A1- 2004 236 285

## Beschreibung

Die vorliegende Erfindung bezieht sich auf eine Dosiervorrichtung für eine Injektionsvorrichtung und insbesondere auf eine Dosiervorrichtung, mit welcher eine Dosis oder Menge einer aus oder mit Hilfe der Injektionsvorrichtung abzugebende Substanz eingestellt werden kann.

Aus der DE 202 09 051 U1 ist ein Injektionsgerät mit endpositionsblockiertem Dosiseinstellglied bekannt, wobei das Injektionsgerät ein Dosiseinstellglied aufweist, das für die Auswahl der Produktdosis relativ zu dem Gehäuse eine rotatorische Bewegung in eine erste Drehrichtung bis in eine Endposition und in eine entgegengesetzte Drehrichtung ausführen kann und das mit einer Fördereinrichtung, mit welcher eine ausgewählte Produktdosis aus einem Reservoir ausschüttbar ist, so gekoppelt ist, dass die Fördereinrichtung bei einer Betätigung die mittels des Dosiseinstellgliedes ausgewählte Produktedosis ausschüttet. Eine Verdrehsicherungseinrichtung verhindert eine rotatorische Bewegung des Dosiseinstellgliedes in die erste Drehrichtung über eine Endposition hinaus.

Die EP 0 828 527 B1 offenbart ein Injektionsgerät mit einer längsverschiebbaren Vorschubhülse, wobei ein Dosisaufdruck zum Ablesen einer zu injizierenden Dosis vorgesehen ist und ein Mechanismus vorgesehen ist, der ein Laden des Injektionsgeräts durch Herausziehen einer Schubstange dann verhindert, wenn der Vorrat einer Ampulle vollständig aufgebraucht ist, wobei die Dosiervorrichtung eine tatsächlich mögliche verabreichbare Dosis an einer im Bereich eines oberen Endes der Vorschubhülse angebrachten Dosisskala ablesbar ist.

Aus der deutschen Patentanmeldung Nr. 10 2005 001 159.4 ist eine Dosiervorrichtung für eine Injektionsvorrichtung mit Übersetzung bekannt, mit welcher insbesondere eine kleine Dosismenge exakt eingestellt und ausgeschüttet werden kann.

Die WO 2004/078239 A1 offenbart eine Medikamentenverabreichungsvorrichtung mit einem Gehäuse mit Innengewinde, einer Dosiswählhülse mit einem Gewinde, welches in das Innengewinde des Gehäuses eingreift, einer Drehhülse, welche lösbar mit der Dosiswählhülse verbunden ist, und einer Kupplung, welche zwischen der Dosiswählhülse und der Drehhülse angeordnet ist, wobei sich beide Hülsen in Bezug auf das Gehäuse drehen können, wenn die Dosiswählhülse und die Drehhülse gekoppelt sind. Wenn die Dosiswählhülse und die Drehhülse entkoppelt sind, wird eine Rotation der Dosiswählhülse in Bezug auf das Gehäuse ermöglicht, während eine Drehung der Drehhülse in Bezug auf das Gehäuse gesperrt ist, wodurch eine axiale Bewegung der Drehhülse ermöglicht wird, so dass eine Kraft in die longitudinale Richtung zu dem proximalen Ende der Medikamentenverabreichungsvorrichtung übertragen wird.

Die DE 10 2005 023 824 A1 offenbart eine Dosiervorrichtung zur Einstellung einer aus einer Injektionsvorrichtung abzugebenden Dosis mit einem Einstellelement, insbesondere einer Einstellhülse, und einem Aufziehelement, insbesondere einer Drehhülse, das aus der Dosiervorrichtung zur Vorbereitung einer Dosisabgabe herausgedreht werden kann, wobei auf dem Einstellelement ein Anschlag und auf dem Aufziehelement ein Gegenanschlag angebracht ist, so dass eine Drehbewegung des Aufziehelements relativ zum Einstellelement durch die Anschläge begrenzt werden kann; sowie ein Verfahren zum Einstellen einer Dosis, welche aus einer Injektionsvorrichtung abgegeben werden soll, wobei die abzugebende Dosis mit einem Einstellelement, insbesondere einer Einstellhülse, eingestellt werden kann und anschließend die Injektionsvorrichtung mit einem Ausziehelement, insbesondere einer Drehhülse, durch eine Drehbewegung aufgezogen werden kann, um in einem anschließenden Schritt die eingestellte Dosis aus der Injektionsvorrichtung abzugeben, wobei der Einstellvorgang von dem Aufziehvorgang entkoppelt ist.

Aus der WO 02/053214 A1 ist eine Dosiseinstellungsvorrichtung zur Verwendung in Kombination mit einem fluidgefüllten Behälter bekannt, wobei die Dosiseinstellungsvorrichtung auf eine wiederholte Injektion von einzeln eingestellten Fluiddosen aus dem Behälter angepasst ist, wobei die Dosiseinstellungsvorrichtung umfasst: ein Gehäuse; ein Antriebselement, das mit dem Gehäuse verbunden und auf den Ausstoß einer Fluiddosis aus dem Behälter angepasst ist; ein im Gehäuse angebrachtes Federelement; ein Dosiseinstellungssystem, das im Gehäuse angebracht und an das Federelement angeschlossen ist, wobei das Dosiseinstellungssystem ein Dosiseinstellungselement umfasst, das in einer ersten Richtung zu einer ausgewählten Einstellungsposition gegen die Vorspannung des Federelements beweglich ist, wobei die Bewegung des Dosiseinstellungselements vom Spannen der Feder begleitet ist; ein auslösbares Sperrelement, das mit dem Gehäuse verbunden und auf das Halten des Dosiseinstellungselements in der eingestellten Position gegen die Vorspannung des Federelements angepasst ist, wobei das Auslösen des Sperrelements bewirkt, dass das Dosiseinstellungssystem das Antriebselement antreibt, um dadurch eine eingestellte Dosis aus einem fluidgefüllten Behälter auszustoßen, wenn die Dosiseinstellungsvorrichtung in Kombination damit verwendet wird, wobei die Kraft zum Ausstoßen der eingestellten Dosis durch das Federelement bereitgestellt wird; wobei das Dosiseinstellungselement in einer zweiten Richtung beweglich ist, um die Einstellungsposition wahlweise einzustellen.

Weitere Dosiervorrichtungen sind aus den Dokumenten US 5 298 023 A, US 2004/236285 A1 und EP0295075 A bekannt geworden.

Die WO 2006/089734 A offenbart eine Dosiervorrichtung mit den Merkmalen des einleitenden Teils des Anspruchs 1.

Es ist eine Aufgabe der vorliegenden Erfindung eine Dosiervorrichtung zur Einstellung und / oder Abgabe einer aus einer Injektionsvorrichtung abzugebenden Dosis, sowie eine Injektionsvorrichtung mit einer solchen Dosiervorrichtung, vorzuschlagen, mit welchen eine bequeme und korrigierbare Einstellung der abzugebenden Dosis ermöglicht wird.

Diese Aufgabe wird durch den unabhängigen Anspruch 1 gelöst, wobei sich vorteilhafte Ausführungsformen aus den abhängigen Ansprüchen ergeben.

Eine erfindungsgemäße Dosiervorrichtung für eine Injektionsvorrichtung zur Einstellung einer aus der Injektionsvorrichtung abzugebenden Dosis einer zum Beispiel in der Injektionsvorrichtung oder einer Ampulle enthaltenen zum Beispiel medizinischen Substanz, wie zum Beispiel Insulin oder Hormone, weist eine Gewindestange auf, welche zum Abgeben einer Dosis in die Injektionsvorrichtung, zum Beispiel geführt durch ein Innengewinde der Injektionsvorrichtung, gedreht werden kann. Bezüglich einer möglichen Ausführungsform einer solchen Gewindestange und einer Injektionsvorrichtung wird auf die DE 10 2005 023 824 A1 der Anmelderin verwiesen. Auf der Gewindestange ist ein Halteelement vorgesehen, welche die Gewindestange gegen ein Verdrehen in eine oder beide Richtungen sichert. Beispielsweise kann das Gewinde der Gewindestange gezahnt sein, wie zum Beispiel in der DE 10 2005 023 824 gezeigt. Ebenso ist es auch möglich, dass nicht auf dem Außengewinde der Gewindestange liegende Haltelemente, wie beispielsweise ein ringförmiger oder umlaufender gezahnter Bereich, auf der Gewindestange vorgesehen sind, um die Gewindestange in einer Drehposition zu halten und gegen eine Verdrehung in mindestens eine Richtung zu sichern, wobei die Gewindestange je nach Eingriffselement, das in das oder die Haltelemente eingreift, in eine der Halterichtung entgegengesetzten Richtung gedreht werden kann.

Ein Einstellelement, wie zum Beispiel eine Drehhülse (rotating sleeve), ist so an oder auf der Gewindestange angeordnet, dass das Einstellelement so relativ zur Gewindestange gedreht werden kann, dass sich das Lageverhältnis zwischen dem Einstellelement und der Gewindestange zum Beispiel durch einen Einstellvorgang verändern lässt, wie zum Beispiel in der DE 10 2005 023 824 beschrieben. Dabei wird die Gewindestange durch ein Halteelement, welches beispielsweise mit der Injektionsvorrichtung oder einer später beschriebenen Führungshülse (guiding sleeve) verbunden ist, gehalten, wodurch es möglich ist, dass das Einstellelement, wie zum Beispiel die Drehhülse, um die Gewindestange gedreht wird, um die Menge einer nach Beendigung des Einstellvorgangs und Auslösung einer Injektion abzugebenden Substanz einzustellen. Dabei kann das Einstellelement relativ zur Gewindestange gedreht und auch in axialer Richtung der Gewindestange verschoben werden, was zum Beispiel dadurch realisiert werden kann, dass das Einstellelement oder die Drehhülse ein Innengewinde hat, in welches das Außengewinde der Gewindestange eingreift. Alternativ ist es möglich, dass die Drehhülse nur relativ zur Gewindestange verdreht wird ohne in axiale Richtung verschoben zu werden.

Vorzugsweise ist das Einstellelement, welches beispielsweise als Drehhülse ausgeführt ist, mit einem Federelement gekoppelt, welches zum Beispiel durch die Drehung des Einstellelementes gespannt werden kann und die zur Abgabe der Substanz erforderliche Energie aufnimmt oder speichert, welche nach Freigabe der Gewindestange von dem Einstellelement bzw. der Drehhülse auf die Gewindestange übertragen wird, so dass sich diese in distale Richtung der Injektionsvorrichtung bewegt, wenn die Gewindestange in einem Innengewinde der Injektionsvorrichtung geführt wird, um die Drehbewegung der Drehhülse in eine Vorschubbewegung der Gewindestange umzusetzen.

Ein Haltelement der Dosiervorrichtung, wie zum Beispiel eine Führungshülse (guiding sleeve), ist vorzugsweise fest mit der Injektionsvorrichtung verbunden und weist mindestens ein Halteelement, in Form von einem Rastarm oder Schnapparm auf, welche in Halteelemente, wie zum Beispiel einen gezahnten umlaufenden Ring oder in ein gezahntes Gewinde der Gewindestange, eingreifen und eine Drehbewegung der Gewindestange verhindern. Wird die Kopplung zwischen dem Halteelement und der Gewindestange zum Beispiel mittels Eindrücken eines Auslöseknopfes gelöst, so kann die Gewindestange, an welcher zum Beispiel die Federkraft des durch die Einstellbewegung aufgezogenen Einstellelementes anliegt, von dem Einstellelement mitgenommen und gedreht werden, um die Gewindestange in distale Richtung der Injektionsvorrichtung zu bewegen und so eine Ausschüttung der Substanz zu bewirken.

Die Kopplung des oder der Eingriffselemente des Einstellelementes in die Gewindestange ist so ausgestaltet, dass sich das Einstellelement relativ zur Gewindestange nur in eine Richtung und bevorzugt nur in eine Drehrichtung bewegen kann, wohingegen eine Bewegung oder Drehung des Einstellelementes in entgegengesetzte Richtung durch die in das oder die Haltelemente der Gewindestange eingreifende Eingriffselemente des Einstellelementes verhindert wird. Das Einstellelement ist somit bezüglich einer Sperrrichtung verdrehgesichert mit der Gewindestange gekoppelt, wohingegen eine Drehung des Einstellelementes relativ zur Gewindestange in entgegengesetzter Richtung, einer Dreh- oder Freigaberichtung, ermöglicht wird. Diese Kopplung wird durch eine Zahnung der Gewindestange und das Eingriffselement realisiert, welche beispielsweise eine flache und eine steile Flanke aufweisen.

Erfindungsgemäß ist diese Kopplung zwischen Einstellelement und Gewindestange während eines Einstellvorganges lösbar, um eine Dosiskorrektur durch Zurückdrehen der Drehhülse vornehmen zu können, falls eine zu hohe Dosis eingestellt wurde. Hierzu kann beispielsweise ein zum Beispiel verschieb- oder verdrehbares Freistellelement vorgesehen sein, welches die Kopplung zwischen dem Einstellelement und der Gewindestange aufhebt. Beispielsweise kann ein parallel zur Gewindestange verschiebbares Freistellelement mit bevorzugt abgeschrägter Fläche vorgesehen sein, welches, wenn es in den Bereich der Kopplung zwischen dem Einstellelement und der Gewindestange eingeschoben wird, mittels der schrägen Fläche die Kopplung des Einstellelementes mit der Gewindestange, durch Anheben oder Wegdrücken der Eingriffselemente, welche zum Beispiel als Anlagefläche für das Freistellelement auch eine Abschrägung aufweisen, löst, so dass das Einstellelement relativ zur Gewindestange frei bewegbar oder in jede Richtung frei drehbar ist. Nach Herausschieben des Freigabeelementes kann das Einstellelement in der zum Beispiel zur Dosiskorrektur korrigierten Position oder Drehposition wieder mit der Gewindestange gekoppelt werden.

Auf der gezahnten Gewindestange ist somit ein Einstellelement, beispielsweise eine Drehhülse vorgesehen, welches zum Einstellen einer Dosis in eine Richtung verdreht werden kann, wobei sich das Einstellelement bevorzugt selbsttätig gegen ein Zurückdrehen sperrt. Diese Selbsthemmung des Einstellelementes, beispielsweise als Drehhülse ausgeführt, kann zum Beispiel dadurch aufgehoben werden, dass das selbsthemmende Element der Drehhülse angehoben wird, also zum Beispiel dadurch, dass der Eingriff der Drehhülse in die Zahnung der Zahngewindestange gelöst wird, wodurch ein Zurückdrehen der Drehhülse zur Dosiskorrektur ermöglicht wird.

Das Freigabeelement zum Entkoppeln des Einstellelementes von der Gewindestange kann auch Bestandteil des Auslöseknopfes sein, welcher beispielsweise ein bis zur Kopplung zwischen dem Einstellelement und der Gewindestange hineinragendes Freigabeelement aufweist, welches bereits durch leichtes Eindrücken des Auslöseknopfes eine Entkopplung des Einstellelementes von der Gewindestange ermöglicht. Dabei ist das Freigabeelement vorzugsweise so angeordnet, dass die Entkopplung des Einstellelementes von der Gewindestange realisiert werden kann, ohne dass eine Auslösung, also die Freigabe der Gewindestange durch das die Gewindestange haltende Halteelement, erfolgt.

Die erfindungsgemäße Dosiervorrichtung ermöglicht somit eine einfache Dosiskorrektur, zum Beispiel bei einer in einem Einstellelement geführten Gewindestange, wobei die Menge der abzugebenden Substanz durch das in einer Ausgangsstellung eine Drehbewegung nur in eine Richtung ermöglichende Einstellelement festgelegt wird, was einen einfachen Aufbau des Einstellelementes ermöglicht. Eine beim Dosieren zuviel eingestellte Dosis kann nach Entkopplung des Einstellelements von der Gewindestange mittels des Freistellelements durch Zurückdrehen des Einstellelementes korrigiert werden, ohne dass dabei eine Ausschüttung erfolgt.

Gemäß einer nicht erfindungsgemäßen Ausführungsform ist das Einstellelement oder die Drehhülse auf der Gewindestange oder der Zahngewindestange während des Einstellvorganges frei drehbar, wobei vorzugsweise kein Eingriffselement, wie zum Beispiel ein Schnapparm der Drehhülse, in die Gewindestange eingreift, so dass eine freie Einstellbewegung, wie zum Beispiel eine freie Vor- und Rückdrehbewegung, möglich ist. Erst bei fertig eingestellter und eventuell korrigierter Dosis wird die Kopplung der Drehhülse mit der Gewindestange hergestellt. Dies kann zum Beispiel dadurch erfolgen, dass mittels eines verschiebbaren Ringes ein Halte- oder Rastelement in Eingriff mit einem entsprechenden Gegen-Rastelement der Drehhülse und der Gewindestange gebracht wird, so dass nach der Verschiebung des Ringes eine verdrehgesicherte Kopplung zwischen dem Einstellelement und der Gewindestange möglich ist.

Alternativ zu einer solchen Verdrehsicherung kann die Drehhülse auch so nach Abschluss der Einstellbewegung mit der Gewindestange gekoppelt werden, dass eine Bewegung des Einstellelementes oder der Drehhülse relativ zur Gewindestange nur mehr in eine Richtung möglich ist.

Vorzugsweise wird die Kopplung zwischen dem Einstellelement und der Gewindestange durch das Eingriffselement realisiert, ausgeführt als zum Beispiel ein radial nach innen vorgespannter Schnapparm, welcher so ausgebildet ist, dass im Zusammenwirken mit entsprechenden Gegen- oder Halteelementen oder der Zahnung der Gewindestange entweder ein vollständiges Halten der Gewindestange, also zum Beispiel eine Verdrehsicherung gegen eine Verdrehung in beide Richtungen, oder eine Verdrehsicherung nur gegen eine Richtung, wie zum Beispiel im Sinne einer an sich bekannten Ratsche realisiert werden kann.

Ein wie oben erwähntes verschiebbares und zum Beispiel in axialer Richtung der Gewindestange bewegbares Eingriffselement, um eine Kopplung zwischen dem Einstellelement und der Gewindestange zur realisieren, ist Bestandteil des Einstellelementes.

Gemäß einem weiteren Aspekt bezieht sich die Erfindung auf eine Injektionsvorrichtung mit einer wie oben beschriebenen Dosiervorrichtung.

Die Erfindung wird nachfolgend anhand eines Ausführungsbeispieles beschrieben werden. Es zeigen:
- Figur 1: eine perspektivische Ansicht der Dosiervorrichtung;
- Figur 2: eine Querschnittsansicht der in Figur 1 gezeigten Dosiervorrichtung;

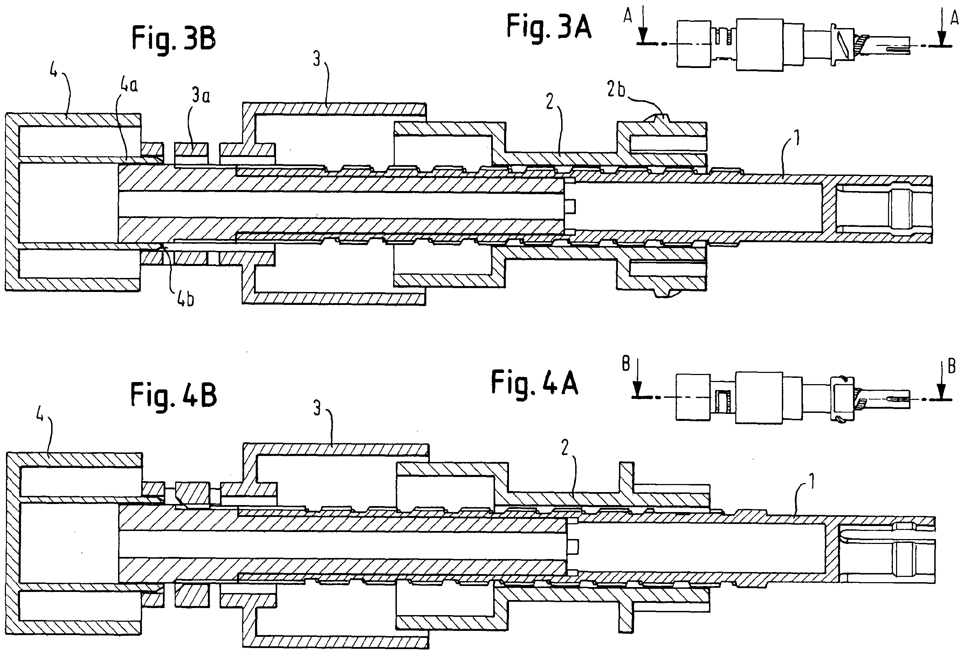

- Figur 3A: eine Draufsicht auf die Dosiervorrichtung;
- Figur 3B: eine Querschnittsansicht entlang der Linie A-A in Figur 3A;
- Figur 4A: eine Draufsicht auf die in Figur 3A gezeigte um 90° gedrehte Dosiervorrichtung;
- Figur 4B: eine Querschnittsansicht entlang der Linie B-B in Figur 4A; und
- Figur 5: eine perspektivische Ansicht einer weiteren Ausführungsform einer Dosiskorrekturvorrichtung.

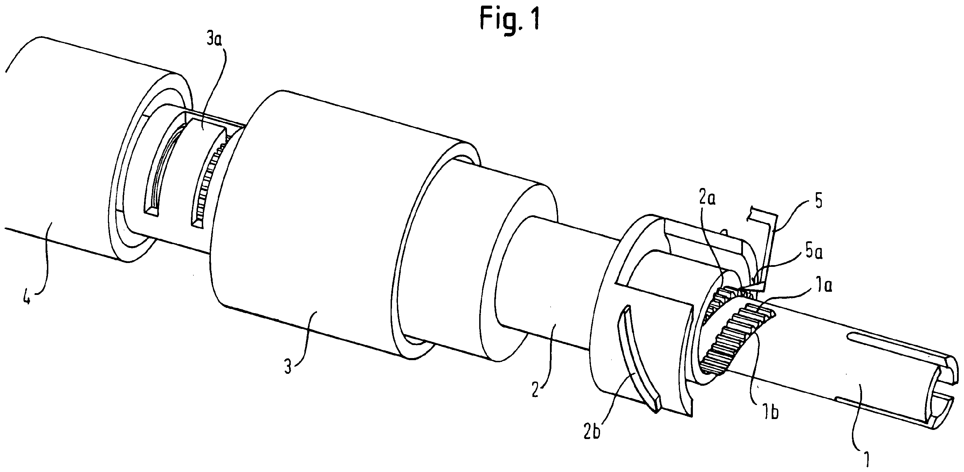

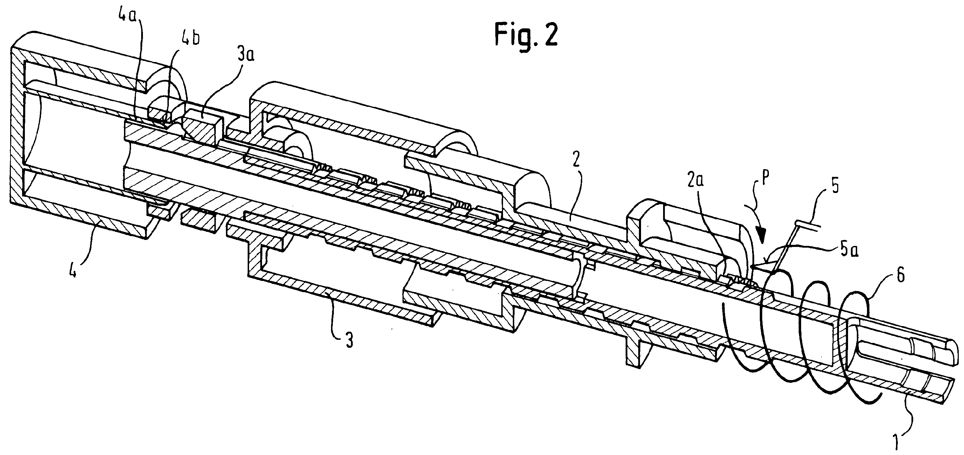

Figur 1 zeigt eine Zahn-Gewindestange 1 mit einem umlaufenden, eine Zahnung 1a tragenden Gewinde 1b. Um die Gewindestange 1 ist als Einstellelement eine Drehhülse 2 vorgesehen, welche Eingriffselemente 2a aufweist, wie auch in Figur 2 gezeigt, die in die Zahnung 1a der Gewindestange 1 eingreifen und eine Drehung der Drehhülse 2 relativ zur Gewindestange 1 in eine durch einen Pfeil P in Figur 2 gezeigte Richtung ermöglichen und eine Drehung in Gegenrichtung blockieren.

Eine Führungshülse 3 ist um die Gewindestange 1 herum angeordnet und weist einen Schnapparm 3a auf, welcher eine Drehung der Gewindestange 1 verhindert.

Das Außengewinde 1b der Gewindestange 1 wird in einem nicht gezeigten Innengewinde der Injektionsvorrichtung geführt.

Wird mittels einer Drehung der Drehhülse 2, welche in der gezeigten Ausführungsform ein Außengewinde 2b aufweist, das in ein korrespondierendes Innengewinde der Injektionsvorrichtung eingreifen kann und somit für eine Verschiebung der Drehhülse 2 in axiale Richtung der Injektionsvorrichtung während eines Einstellvorganges sorgt, eine zu große Dosis eingestellt, so kann mittels eines schematisch dargestellten Freigabeelementes 5, das eine angeschrägte Oberfläche 5a aufweist und so zwischen die Drehhülse 2 und die Gewindestange 1 eingeschoben werden kann, um den Eingriff des Eingriffelementes 2a in die Gewindestange 1 zu lösen, die Drehhülse 2 von der Gewindestange 1 entkoppelt werden, um den zuviel eingestellte Dosis wieder zurückzudrehen und somit eine Dosiskorrektur vorzunehmen. Dabei kann die Drehhülse 2 an ihrer Vorderseite eine korrespondierende Abschrägung aufweisen, um das Einschieben des Freigabeelements 5 und das Anheben und Entkoppeln zu erleichtern. Das Freigabeelement 5 kann z.B. als von außen betätigbares oder verschiebbares Element oder auch als verschiebbare Hülse ausgebildet sein.

Zur Auslösung der Injektion kann die Kopplung zwischen der als Haltelement dienenden Führungshülse 3 und der Zahnstange 1 durch Einschieben des Auslöseknopfes 4 in Richtung der Führungshülse 3 gelöst werden. Hierzu weist der Auslöseknopf 4 in axiale Richtung vorstehende Freigabeelemente 4a mit schrägen Vorderflächen 4b auf, welche an korrespondierende schräge Gegenflächen der Schnapparme 3a der Führungshülse 3 in Anlage kommen können und bei einem weiteren Einschieben des Auslöseknopfes 4 den oder die Schnapparme 3a anheben, also in radiale Richtung weg von der Gewindestange 1 nach außen bewegen, um so die Drehsicherung oder das Halten der Gewindestange 1 zu lösen.

Die von der Führungshülse 3 nicht mehr gehaltene Gewindestange 1 wird nun von der Drehhülse 2 mitgenommen, welche von der während des Einstellvorganges gespannten Feder 6, die sich nun wieder entspannen kann, zurückgedreht wird, was zu einer Drehbewegung der Gewindestange 1 und somit mittels der Kopplung über das Gewinde 1b der Gewindestange 1 in einem Innengewinde der Injektionsvorrichtung zu einer Bewegung der Gewindestange 1 in distale Richtung der Injektionsvorrichtung führt, um die mittels der Aufzieh-Drehbewegung der Drehhülse 2 eingestellte Dosis abzugeben.

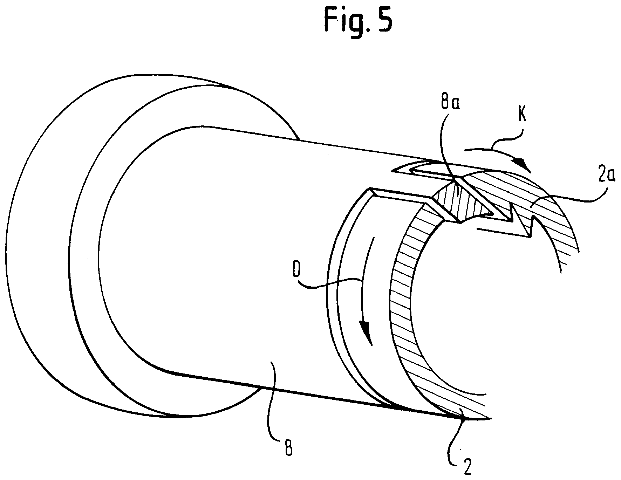

Figur 5 zeigt eine weitere Ausführungsform, wobei die Dosiseinstellung mittels einer Dosierhülse 8 erfolgt, die einen oder mehrere Schnapparmheber 8a aufweist, der so in die Drehhülse 2 eingreift oder mit den Schnapparmen 2a zusammenwirkt, dass eine Drehung in Dosiereinrichtung (Pfeil D) übertragen wird, wobei eine Drehung in Korrekturrichtung (Pfeil K) zu einem Anheben der Schnapparme 2a durch die Abschrägung des Schnapparmhebers 8a führt. Die Schnapparme 2a sind somit von der Gewindestange 1 entkoppelt.

## Patentansprüche

1. Dosiervorrichtung für eine Injektionsvorrichtung zur Einstellung einer aus einer Injektionsvorrichtung abzugebenden Dosis mit einer Gewindestange (1) und mit einem Einstellelement (2), welches relativ zur Dosiervorrichtung gedreht werden kann und welches ein Eingriffselement (2a) aufweist, um so mit einer Zahnung (1a) der Gewindestange (1) gekoppelt zu werden, dass das Einstellelement (2) relativ zur Gewindestange (1) in eine Richtung bewegt werden kann, um eine abzugebende Dosis einzustellen und eine Relativbewegung in Gegenrichtung gesperrt wird, um die abzugebende Dosis auszuschütten; und mit einem Halteelement (3), welches einen Rastarm (3a) oder Schnapparm (3a) aufweist, um eine Drehbewegung der Gewindestange (1) in beide Richtungen oder in eine Richtung durch Zusammenwirken mit entsprechenden Gegen- oder Halteelementen oder der Zahnung der Gewindestange (1) zu blockieren, wobei bei Ausschüttung der abzugebenden Dosis die Koppelung zwischen dem Rastarm (3a) oder Schnapparm (3a) des Halteelements (3) und den entsprechenden Gegen- oder Halteelement oder der Zahnung der Gewindestange (1) gelöst ist, **gekennzeichnet durch** ein Freistellelement (5), mit welchem die Kopplung zwischen dem Einstellelement (2) und der Gewindestange (1) durch Anheben oder Wegdrücken des Eingriffselements (2a) mittels des Freistellelements (5) gelöst werden kann, wobei das Freistellelement (5), wenn es in den Bereich der Koppelung zwischen dem Einstellelement (2) und der Gewindestange (1) geschoben wird, die Kopplung löst, wodurch ein Zurückdrehen des Einstellelements (2) zur Dosiskorrektur ermöglicht wird.

2. Dosiervorrichtung nach Anspruch 1, wobei als Freistellelement (5) ein parallel zur Gewindestange (1) verschiebbares Element mit bevorzugt abgeschrägter Fläche vorgesehen ist.

3. Dosiervorrichtung nach einem der vorhergehenden Ansprüche, wobei nach Herausschieben des Freistellelements (5) das Einstellelement (1) in der korrigierten Position oder Drehposition wieder mit der Gewindestange (1) gekoppelt werden kann.

4. Dosiervorrichtung nach einem der vorhergehenden Ansprüche, wobei das mindestens eine Eingriffselement (2a) einen elastischen Bereich aufweisen.

5. Dosiervorrichtung nach einem der vorhergehenden Ansprüche, wobei das mindestens eine Eingriffselement (2a) einen Rast- oder Schnapparm bildet.

6. Dosiervorrichtung nach einem der vorhergehenden Ansprüche mit einer Feder (6), welche mit dem Einstellelement (2) verbunden und durch eine Einstellbewegung des Einstellelements (2) gespannt werden kann.

7. Dosiervorrichtung nach einem der vorhergehenden Ansprüche mit einem Auslöseelement (4), mit welchem die Kopplung zwischen dem Halteelement (3) und der Gewindestange (1) gelöst werden kann.

8. Dosiervorrichtung nach dem vorhergehenden Anspruch, wobei das Auslöseelement (4) ein Element (4a) mit einer relativ zur radialen Ebene abgeschrägten Fläche aufweist.

9. Dosiervorrichtung nach einem der vorhergehenden Ansprüche, wobei das Einstellelement (2) ein Aussengewinde (2b) aufweist, welches in ein Innengewinde einer Injektionsvorrichtung eingreifen kann.

10. Dosiervorrichtung nach einem der vorhergehenden Ansprüche, wobei die Gewindestange (1) ein Aussengewinde (1b) mit der Zahnung aufweist.

11. Dosiervorrichtung nach einem der vorhergehenden Ansprüche, wobei die Gewindestange (1) einen umlaufenden Bereich (1c) aufweist, an welchem die Zahnung vorgesehen sind.

12. Injektionsvorrichtung mit einer Dosiervorrichtung nach einem der vorhergehenden Ansprüche.

## Claims

1. A dosing device for an injection device for setting a dose to be dispensed from an injection device comprising a threaded rod (1) and a setting element (2) which can be rotated relative to the dosing device and which has an engagement element (2a) in order to be coupled to a tooth arrangement (1a) of the threaded rod (1) in such a way that the setting element (2) can be moved relative to the threaded rod (1) in a direction in order to set a dose to be dispensed and a relative movement in the opposite direction is blocked in order to expel the dose to be dispensed; and a holding element (3) having a latching arm (3a) or snap-engagement arm (3a) to block a rotary movement of the threaded rod (1) in both directions or in one direction by cooperation with corresponding counterpart or holding elements or the tooth arrangement of the threaded rod (1), wherein upon expelling the dose to be dispensed the coupling between the latching arm (3a) or snap-engagement arm (3a) of the holding element (3) and the corresponding counterpart or holding element or the tooth arrangement of the threaded rod (1) is released, **characterised by** a release element (5) with which the coupling between the setting element (2) and the threaded rod (1) can be released by lifting or pressing away the engagement element (2a) by means of the release element (5), wherein when the release element (5) is pushed into the region of the coupling between the setting element (2) and the threaded rod (1) the release element (5) releases the coupling whereby return rotation of the setting element (2) for dose correction is made possible.

2. A dosing device according to claim 1 wherein an element which is displaceable parallel to the threaded rod (1) and has a preferably inclined surface is provided as the release element (5).

3. A dosing device according to one of the preceding claims wherein after the release element (5) has been pushed out the setting element (1) can be coupled to the threaded rod (1) again in the corrected position or rotary position.

4. A dosing device according to one of the preceding claims wherein the at least one engagement element (2a) has an elastic region.

5. A dosing device according to one of the preceding claims wherein the at least one engagement element (2a) forms a latching or snap-engagement arm.

6. A dosing device according to one of the preceding claims comprising a spring (6) which can be connected to the setting element (2) and can be stressed by a setting movement of the setting element (2).

7. A dosing device according to one of the preceding claims having a releasing element (4) with which the coupling between the holding element (3) and the threaded rod (1) can be released.

8. A dosing device according to the preceding claim wherein the releasing element (4) has an element (4a) with a surface inclined relative to the radial plane.

9. A dosing device according to one of the preceding claims wherein the setting element (2) has a male thread (2b) which can engage into a female thread of an injection device.

10. A dosing device according to one of the preceding claims wherein the threaded rod (1) has a male thread (1b) with the tooth arrangement.

11. A dosing device according to one of the preceding claims wherein the threaded rod (1) has a peripherally extending region (1c) at which the tooth arrangement is provided.

12. An injection device having a dosing device according to one of the preceding claims.

## Revendications

1. Dispositif de dosage pour un dispositif d'injection servant à régler une dose à distribuer depuis un dispositif d'injection avec une tige filetée (1) et avec un élément de réglage (2), lequel peut être tourné par rapport au dispositif de dosage et lequel présente un élément de prise (2a) pour être couplé à une denture (la) de la tige filetée (1) de telle manière que l'élément de réglage (2) peut être déplacé dans une direction par rapport à la tige filetée (1) pour régler une dose à distribuer et un déplacement relatif est bloqué dans le sens opposé pour déverser la dose à distribuer ; et avec un élément de maintien (3), lequel présente un bras d'enclenchement (3a) ou un bras à déclic (3a) pour bloquer un déplacement de rotation de la tige filetée (1) dans deux directions ou dans une direction par coopération avec des contre-éléments ou éléments de maintien correspondants ou de la denture de la tige filetée (1), dans lequel lors du déversement de la dose à distribuer, le couplage entre le bras d'enclenchement (3a) ou le bras à déclic (3a) de l'élément de maintien (3) et le contre-élément ou l'élément de maintien correspondant ou la denture de la tige filetée (1) est desserré, **caractérisé par** un élément de déblocage (5), avec lequel le couplage entre l'élément de réglage (2) et la tige filetée (1) peut être desserré en relevant ou en éloignant par pression l'élément de prise (2a) au moyen de l'élément de déblocage (5), dans lequel l'élément de déblocage (5) desserre le couplage quand il est glissé dans la zone du couplage entre l'élément de réglage (2) et la tige filetée (1), ce qui permet un retour par rotation de l'élément de réglage (2) aux fins de la correction de dose.

2. Dispositif de dosage selon la revendication 1, dans lequel est prévu, en tant qu'élément de déblocage (5), un élément pouvant être coulissé de manière parallèle par rapport à la tige filetée (1), avec une surface de manière préférée chanfreinée.

3. Dispositif de dosage selon l'une quelconque des revendications précédentes, dans lequel après la sortie par glissement de l'élément de déblocage (5), l'élément de réglage (1) peut être couplé dans la position corrigée ou la position de rotation à nouveau à la tige filetée (1).

4. Dispositif de dosage selon l'une quelconque des revendications précédentes, dans lequel l'au moins un élément de prise (2a) présente une zone élastique.

5. Dispositif de dosage selon l'une quelconque des revendications précédentes, dans lequel l'au moins un élément de prise (2a) forme un bras d'enclenchement ou à déclic.

6. Dispositif de dosage selon l'une quelconque des revendications précédentes, avec un ressort (6), lequel est relié à l'élément de réglage (2) et peut être tendu par un déplacement de réglage de l'élément de réglage (2).

7. Dispositif de dosage selon l'une quelconque des revendications précédentes, avec un élément de déclenchement (4), avec lequel le couplage entre l'élément de maintien (3) et la tige filetée (1) peut être desserré.

8. Dispositif de dosage selon la revendication précédente, dans lequel l'élément de déclenchement (4) présente un élément (4a) avec une surface chanfreinée par rapport au plan radial.

9. Dispositif de dosage selon l'une quelconque des revendications précédentes, dans lequel l'élément de réglage (2) présente un filetage extérieur (2b), lequel peut venir en prise avec un filetage intérieur d'un dispositif d'injection.

10. Dispositif de dosage selon l'une quelconque des revendications précédentes, dans lequel la tige filetée (1) présente un filetage extérieur (1b) avec la denture.

11. Dispositif de dosage selon l'une quelconque des revendications précédentes, dans lequel la tige filetée (1) présente une zone (1c) périphérique, au niveau de laquelle la denture est prévue.

12. Dispositif d'injection avec un dispositif de dosage selon l'une quelconque des revendications précédentes.
